# EUROPEAN PATENT SPECIFICATION

(11) **EP 1 172 652 B1**
(45) Date of publication and mention of the grant of the patent: **10.06.2015**
(21) Application number: 01904530.1
(22) Date of filing: 19.02.2001
(51) Int. Cl.: G01N 33/48, G01N 33/487, A61B 5/00

(54) **Measuring system**
Testsystem
Système de mesure

(30) Priority: 18.02.2000 JP 2000041714
(43) Date of publication of application: 16.01.2002
(73) Proprietor: Panasonic Healthcare Co., Ltd., Ehime 791-0395 (JP); ARKRAY, Inc., Kyoto-shi, Kyoto 601-8045 (JP)
(72) Inventor: MATSUDA, Kouichi, Ehime 792-0009 (JP); WATANABE, Masashi, Ehime 793-0030 (JP); SATO, Yoshiharu, Kyoto-shi, Kyoto 601-8045 (JP); HIRAO, Etsuo, Kyoto-shi, Kyoto 601-8045 (JP)
(74) Representative: Balsters, Robert
(86) International application number: PCT/JP2001/001151
(87) International publication number: WO 2001/061341

(56) References cited:
- GB-A- 2 322 444
- JP-A- 11 510 915
- JP-A- 62 003 649

## Description

### TECHNICAL FIELD

The present invention relates to a measuring system suitable for a biosensor which can accurately, speedily, and easily assay a specific component in various types of a biological sample and, more particularly, to a measuring system which is surely handled by a blind person or a person having a weak eyesight.

### BACKGROUND ART

In recent years, various types of a biosensor which utilize a specific catalytic action held by enzyme are developed and are attempted to be applied to a clinical field, and a biosensor which can speedily and accurately perform measurement is desired.

When a glucose sensor is cited as an example, conventionally, a patient measures plasma by centrifuging blood in order to measure and manage a blood sugar level, which results in a requirement of considerably complicated procedures. Therefore, a sensor which enables to measure with whole blood is desired in these days of a rapid increase in the number of diabetic patients.

There is a simplified form, in which a stick-type support body is provided with a carrier including enzyme that reacts only to sugar (glucose) and a color matter which is changed at enzyme reaction or by a product of the enzyme reaction, similarly as examination paper used at urine examination. This is a method, in which blood is dropped on the carrier to visually or optically measure the change of the color matter after a certain period of time. Though, according to the method, an accuracy becomes low due to large interference by a coloring substance in the blood.

On the other hand, a measuring system which is disposable with an electrode system at every measurement is proposed. Though, while measuring operation is considerably simplified, such a measuring device system becomes very expensive in view of an electrode material such as platinum and the like or a construction. While a sputtering method or a deposition method can be employed as a manufacturing method of platinum electrodes, the manufacturing cost must become very high.

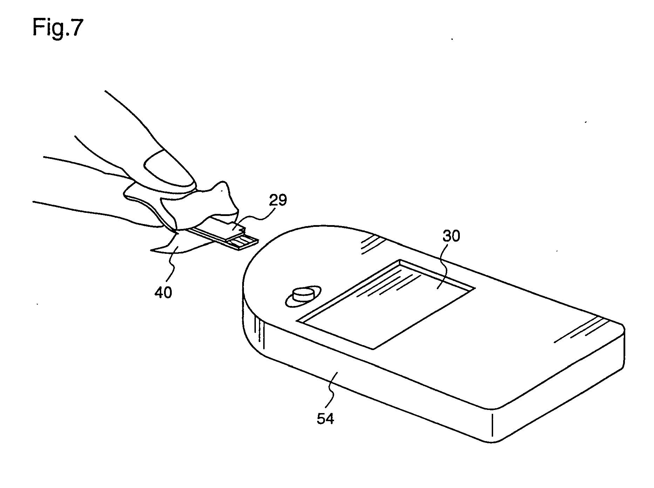

A biosensor disclosed in Japanese Published Patent Application No. Sho. 61-294351 is proposed as a method which enables to make the measuring device disposable with an electrode system. According to these measurements, prescribed voltage is applied to an electrode system of the sensor to measure a value of an electric current flowing between electrodes, and concentration of a substrate in sample liquid is calculated based on the signal. Conventionally, aluminum package 40 to be a packing material for a sensor 29 is opened to a prescribed position, and the sensor is inserted to a connector part of a measuring device 54, which holds the sensor, with pinched with the aluminum package 40, as shown in figure 7. Then, after conforming by a display means 30 that the power is automatically turned on, blood is applied to the edge of the sensor 29, thereby performing a measurement.

EPA-0 762 311 describes a measuring system comprising a sensor and a measuring device for assaying a specific component in a biological sample applied to the sensor, said measuring system being constructed such that a tip or test strip projects farther than a guide member.

According to a measuring system having a conventional construction, a connector part of the measuring device is small and its position is hard to be found, whereby it is difficult to determine a position so as to insert the sensor to the connector part of the measuring device or to apply blood of a fingertip or the like to a prescribed position of the sensor, and particularly, the handling is considerably difficult for a blind person or a person having a weak eyesight.

The present invention is made to solve the above-mentioned problems and has for its object to provide a measuring system in which an insert position of a sensor and an applying position of blood or the like in a measuring device are easy to be found by attaching a guide for a fingertip, thereby easily, speedily, and accurately measuring a specified component in a biological sample such as blood.

### DISCLOSURE OF THE INVENTION

According to claim 1 of the present invention, there is provides a measuring system comprising a sensor and a measuring device for assaying a specified component in a biological sample applied to the sensor, in which a new sensor is mounted to the measuring device at every time when a property of a specific compound in the biological sample is measured, so as to perform a measurement, including: a V-shape guide for guiding a fingertip to which the biological sample is attached, to the sensor to which the biological sample is to be applied, said V-shape guide having a pair of guide pieces each projecting beyond an end portion of said sensor and diverging from each other in an outward direction away from said sensor, so a to define a space broadening in said outward direction so as to guide said fingertip toward said sensor, wherein the guide is provided at a forward side of the measurement part of the sensor.

Therefore, a user such as person having weak sight or an elderly person can easily find the guide position and performs a sliding operation of his/her finger along the guide, thereby easily applying a target to be measured to the sensor.

Consequently, a user such as an elderly person or a person having weak sight can easily perform a measuring operation of a target to be measured, resulting in an easy handling of the system.

According to claim 2 of the present invention, in the measuring system as defined in claim 1, the guide can be pulled out of or stored in the measuring device.

Therefore, a user such as a person having weak sight or an elderly person can easily establish the system, thereby easily performing a measuring operation without any troublesome operations for each measurement.

According to claim 3 of the present invention, in the measuring system as defined in Claim 1, an optional equipment which announces a measurement result by the measuring device by voice is further included, and the guide is mounted to the side of the optional equipment.

Therefore, a user such as a person having weak sight or an elderly person who needs to use the optional equipment can easily establish the system and apply a target to be measured to the sensor.

According to claim 4 of the present invention, in the measuring system as defined in claim 3, the guide is employed as a clamp for fixing the measuring device with the optional equipment.

Therefore, a complicated mechanism is not especially required, whereby a user such as a person having weak sight or an elderly person can easily perform a measuring operation without any bothering operations at every measurement.

According to claim 5 of the present invention, in the measuring system as defined in claim 3, the guide can be pulled out of or stored in the optional equipment.

Therefore, a complicated mechanism is not especially required, whereby a user such as a person having weak sight or an elderly person can easily perform a measuring operation without any bothering operations at every measurement.

According to claim 6 of the present invention, in the measuring system as defined in claim 1, the guide is made of a material having a spring elasticity.

Therefore, even a person having weak sight or an elderly person can easily perform a handling of the guide as a clamp.

According to claim 7 of the present invention, in the measuring system as defined in claim 1, the guide has the space formed from an end of the measuring device.

Therefore, a user such as a person having weak sight or an elderly person performs a sliding operation of his/her finger along the guide, thereby easily applying a target to be measured to the sensor.

According to claim 8 of the present invention, in the measuring system as defined in claim 4, the guide pushes the end or top surface of the measuring device.

Therefore, a complicated mechanism is not especially required, whereby a user such as a person having weak sight or an elderly person can easily perform a measuring operation without any bothering operations at every measurement.

According to claim 9 of the present invention, in the measuring system as defined in claim 3, the guide is removable from the optional equipment.

Therefore, the guide can be easily removed when not needed.

### BRIEF DESCRIPTION OF DRAWINGS

Figure 1 is a perspective view of a measuring system according to an embodiment of the present invention.
Figure 2 is a perspective view of the measuring system seen from the bottom side according to the embodiment of the invention.
Figure 3 is an exploded perspective view of a biosensor used for the measuring system according to the embodiment of the invention.
Figure 4 is a side view illustrating a state where a guide is attached to an optional equipment in the measuring device according to the embodiment of the invention.
Figure 5 is a plan view of a measuring system exemplifying a use of the guide of the measuring system according to the embodiment of the invention.
Figure 6 is a sectional view of a measuring system according to another embodiment of the present invention.
Figure 7 is a perspective view of a conventional measuring device.

### BEST MODE TO EXECUTE THE INVENTION

Hereinafter, a measuring system according to the present invention will be described on the basis of a concrete embodiment.

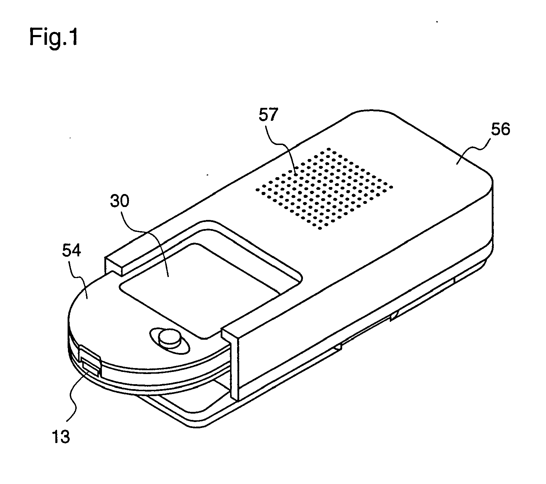

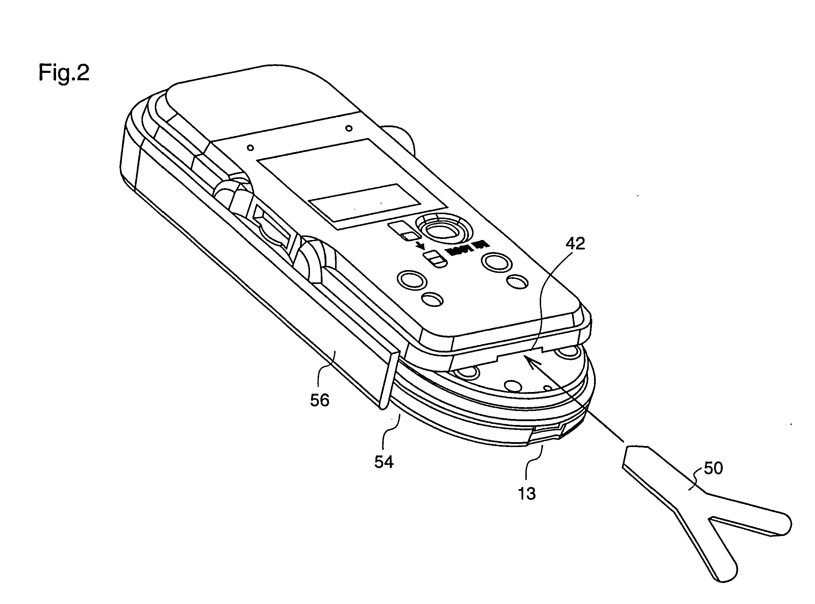

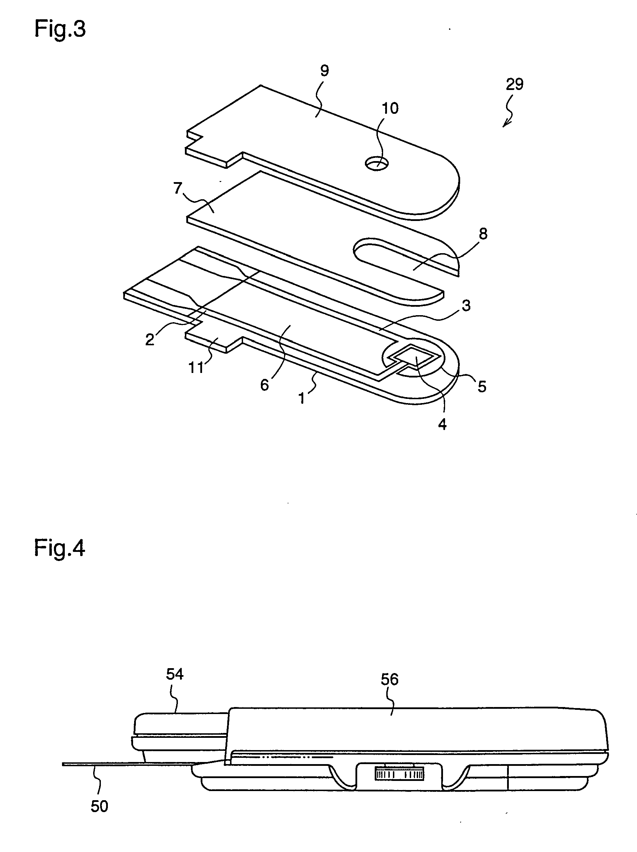

Figure 1 is an external perspective view of the measuring system, figure 2 is a perspective view of the measuring system seen from the bottom side, and figure 3 is an exploded perspective view of a biosensor (hereinafter, abbreviated to sensor).

As shown in figure 1, in a state where a measuring device 54 is united with an optional equipment 56, a sensor 29 shown in figure 3 is inserted to a connector 13, and liquid to be examined such as blood is applied to the end of the sensor 29, so that a measurement result in the measuring device 54 is sent to the optional equipment 56, thereby announcing the measurement result by voice from a voice output means 57 of the optional device 56.

Further, a counter electrode 5, a measuring electrode 4, leads 3 and 2 connected thereto, and an insulating layer 6 are provided on a substrate 1 of the sensor 29 as shown in figure 3. A reactive layer, which is not shown, which includes enzyme and a mediator (electron acceptor) is formed as if the counter electrode 5 and the measuring electrode 4 are covered therewith. A cover 9 is fixed on the substrate 1 through the intermediary of a spacer 7. Numeral 8 denotes a sample supply opening, from where liquid to be examined (sample) is introduced onto the counter electrode 5 and the measuring electrode 4 by capillarity. The air inside is discharged from an air vent 10 together with the introduction of the liquid to be examined. Numeral 11 denotes a projection for preventing an inverse insertion, by which a reverse insertion to the body of the measuring device 54 can be prevented.

A usage example of a guide in the measuring device according to the invention will be described with reference to figures 2, 4, and 5.

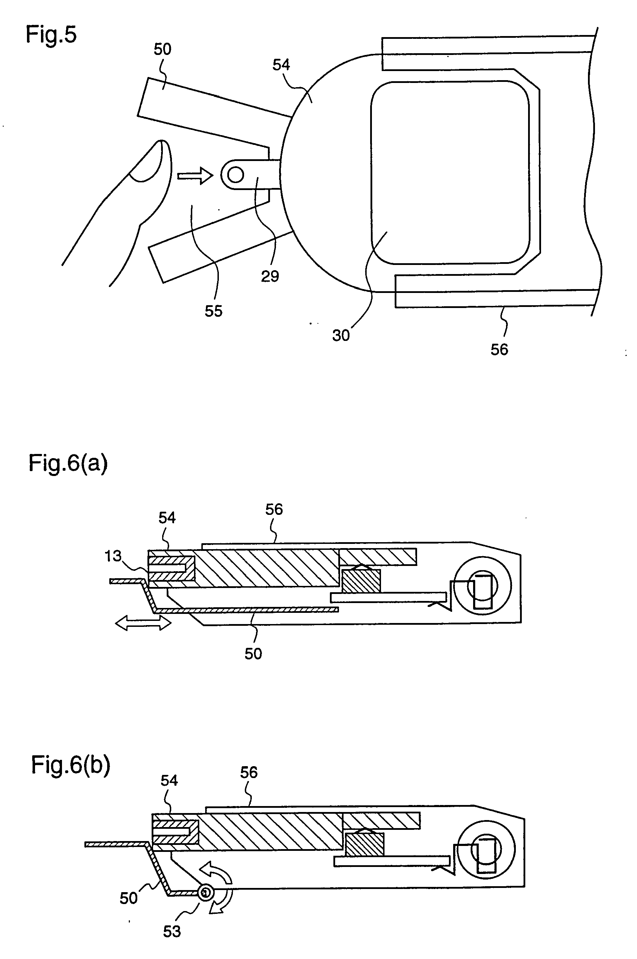

Figure 4 is a side view of the measuring system. Figure 5 is a plan view illustrating a sectional construction of a guide 50 for applying body fluid to the sensor and a sensor hold part in the measuring system.

As shown in figure 2, in a state where the optional equipment 56 is attached to the measuring device 54, the guide 50 is inserted to a guide insertion slot 42 of the optional equipment 56, the sensor 29 is inserted to the connector 13 of the measuring device 54, thereby to employ the guide 50 as a guiding means for a fingertip. After completing a puncture of a fingertip by a lancet, a measurement operator moves his/her fingertip to the end of the sensor 29 along a guiding groove 55 for guiding a fingertip, which is provided in the guide 50, thereby to perform an applying operation of prescribed body fluid, as shown in figure 5. While the guide is V-shape in the embodiment, it may have any other shapes than the V-shape if only there is a groove which leads a fingertip to the end of the measuring device where the sensor is attached.

Thereafter, at the end of the sensor 29, to which body fluid is applied, a prescribed measurement result concerning the body fluid is notified by voice or pressure by an optional equipment 56, or a display means 30 such as a LCD of the measuring device 54 due to progress of an enzyme reaction.

When this measuring operation is completed, the measurement operator confirms the position of the sensor 29 by the guide 50 to be a mark, and concludes the operation by taking the sensor 29 out of the connector 31.

Here, the guide 50 is attached to the optional equipment 56 which is thought to be attached to the measuring device 54 in a case of a usage by a blind person, a person having a weak eyesight or an elderly person, or is insertedly fitted to a fitting part of the measuring device 54 and the optional equipment 56. In case where the guide 50 is insertedly fitted to the fitting part, the guide 50 is in a conformation as shown in above-described figure 4. On the other hand, while in case where the guide 50 is attached to the side of the optional equipment 56, the guide 50 can be used as a clamp for unitedly fixing the measuring device 54 and the optional equipment 56 as shown in figures 6(a) and (b). In figure 6(a), the guide 50 is stored into the optional equipment 56 in an arrow direction by a sliding system, which results in a compact shape. In figure 6(b), the guide 50 can be constructed to rotate around a supporting point 53 of the optional equipment 56. In either case, the end of the guide 50 is inflected, thereby to obtain a clampable shape at the end of the measuring device 54.

As a material of the guide 50, a material especially having a spring elasticity (such as SUS material or Duracon) is employed, so that the guide can be employed as a stronger clamp due to its own spring elasticity.

As described in the above-mentioned embodiment, when the guide 50 is employed, it is possible to remove difficulty of locating operations when a blind person or a person having a weak eyesight attaches/detaches the sensor 29 to/from the measuring device 54, or applies body fluid such as blood of a fingertip to the end of the sensor 29, thereby reducing troubles or labors of the operations at the usage. Further, the mechanism for guiding a target to be measured to the sensor 29 can be realized by a simple construction such as the guide 50.

In the above description, as the sensor 29, a type of a sensor which utilizes capillarity as a method of leading blood to an enzyme electrode part is described. Though, even when a sensor which drops blood directly to the enzyme electrode part can be employed, the same effects can be obtained.

Further, the guide 50 may be constructed to be attached to the measuring electrode 54 directly, in addition to construction as described in the above-mentioned embodiment. For example, the guide 50 may be detachable from the measuring device 54, or may have construction similar to that shown in figure 6, in which the guide 50 may be constructed to be stored into the measuring device 54 by a sliding system, or may be supported to rotate freely.

### APPLICABILITY IN INDUSTRY

As described above, a measuring system according to the present invention can remove difficulty of locating operations when a sensor is attached/detached to/from a measuring device or body fluid such as blood of a fingertip is applied to the end of the sensor, and more particularly, the measuring system is available when its user is a blind person or a person having a weak eyesight, resulting in reduction of troubles or labors of the operation at the usage.

## Claims

1. A measuring system comprising a sensor (29) and a measuring device (54) for assaying a specified component in a biological sample applied to the sensor (29), in which a new sensor is mounted to the measuring device (54) at every time when a property of a specific compound in the biological sample is measured, so as to perform a measurement, including :
a V-shape guide (50) for guiding a fingertip to which the biological sample is attached, to the sensor (29) to which the biological sample is to be applied, said V-shape guide (50) having a pair of guide pieces each projecting beyond an end portion of said sensor (29) and diverging from each other in an outward direction away from said sensor, so as to define a space (55) broadening in said outward direction so as to guide said fingertip toward said sensor (29),
wherein the guide (50) is provided at a forward side of the measurement part of the sensor (29).

2. The measuring system as defined in Claim 1, wherein the guide (50) can be pulled out of or stored in the measuring device (54).

3. The measuring system as defined in Claim 1, further including an optional equipment (56) which announces a measurement result by the measuring device by voice, wherein
said guide (50) is mounted to the side of the optional equipment (56).

4. The measuring system as defined in Claim 3, wherein the guide (50) is employed as a clamp for fixing the measuring device (54) with the optional equipment (56).

5. The measuring system as defined in Claim 3, wherein the guide (50) can be pulled out of or stored in the optional equipment (56).

6. The measuring system as defined in Claim 1, wherein the guide (50) is made of a material having a spring elasticity.

7. The measuring system as defined in Claim 1, wherein the guide (50) has the space (55) formed from an end of the measuring device (54).

8. The measuring system as defined in Claim 4, wherein the guide (50) pushes the end or top surface of the measuring device (54).

9. The measuring system as defined in Claim 3, wherein the guide (50) is removable from the optional equipment (56).

## Patentansprüche

1. Testsystem, aufweisend einen Sensor (29) und eine Messvorrichtung (54) zum Untersuchen einer spezifizierten Komponente in einer biologischen Probe, die beim Sensor (29) angewandt wird, wobei ein neuer Sensor jedes Mal an der Messvorrichtung (54) angebaut wird, wenn eine Eigenschaft einer spezifischen Verbindung in der biologischen Probe gemessen wird, um eine Messung durchzuführen, aufweisend:
eine V-förmige Führung (50) zum Führen einer Fingerspitze, an der die biologische Probe angebracht ist, zum Sensor (29), an dem die biologische Probe anzubringen ist, wobei die V-förmige Führung (50) ein Paar von Führungsteilen aufweist, die jeweils hinter einem Endbereich des Sensors (29) vorstehen und vom Sensor weg auseinanderlaufen und hierdurch einen Raum (55) zu bilden, der sich nach außen hin verbreitert und dadurch die Fingerspitze in Richtung zum Sensor (29) zu führen,
wobei die Führung (50) auf einer vorderen Seite des Messteils des Sensors (29) bereitgestellt ist.

2. Testsystem nach Anspruch 1, wobei die Führung (50) aus der Messvorrichtung (54) herausgezogen oder in dieser aufbewahrt werden kann.

3. Testsystem nach Anspruch 1, ferner aufweisend eine optionale Ausrüstung (56), die ein Messergebnis von der Messvorrichtung durch Sprache bekannt gibt, wobei
die Führung (50) auf der Seite der optionalen Ausrüstung (56) angebaut ist.

4. Testsystem nach Anspruch 3, wobei die Führung (50) als eine Klammer zum Befestigen der Messvorrichtung (54) an der optionalen Ausrüstung (56) angeordnet ist.

5. Testsystem nach Anspruch 3, wobei die Führung (50) aus der optionalen Ausrüstung (56) herausgezogen oder in dieser gespeichert werden kann.

6. Testsystem nach Anspruch 1, wobei die Führung (50) aus einem Werkstoff hergestellt ist, der federnde Elastizität aufweist.

7. Testsystem nach Anspruch 1, wobei bei der Führung (50) der Raum (55) von einem Ende der Messvorrichtung (54) geformt ist.

8. Testsystem nach Anspruch 4, wobei die Führung (50) die End- oder obere Fläche der Messvorrichtung (54) drückt.

9. Testsystem nach Anspruch 3, wobei die Führung (50) von der optionalen Ausrüstung (56) abnehmbar ist.

## Revendications

1. Système de mesure comprenant un capteur (29) et un dispositif de mesure (54) pour doser un composant spécifié dans un échantillon biologique appliqué au capteur (29), où un nouveau capteur est monté sur le dispositif de mesure (54) à chaque fois qu'une propriété d'un composé spécifique dans l'échantillon biologique est mesurée, de manière à effectuer une mesure, comportant :
un guide en forme de V (50) pour guider un bout de doigt auquel l'échantillon biologique est fixé, au capteur (29) auquel l'échantillon biologique doit être appliqué, ledit guide en forme de V (50) ayant une paire de pièces de guidage se projetant chacune au-delà d'une partie d'extrémité dudit capteur (29) et s'écartant l'une de l'autre dans une direction vers l'extérieur loin dudit capteur, de manière à définir un espace (55) s'élargissant dans ladite direction vers l'extérieur de manière à guider ledit bout de doigt vers ledit capteur (29),
dans lequel le guide (50) est prévu au niveau d'un côté avant de la partie de mesure du capteur (29).

2. Système de mesure tel que défini dans la revendication 1, dans lequel le guide (50) peut être retiré du dispositif de mesure (54) ou stocké dans celui-ci.

3. Système de mesure tel que défini dans la revendication 1, comportant en outre un équipement facultatif (56) qui annonce un résultat de mesure par le dispositif de mesure vocalement, dans lequel
ledit guide (50) est monté sur le côté de l'équipement facultatif (56).

4. Système de mesure tel que défini dans la revendication 3, dans lequel le guide (50) est utilisé comme une pince pour fixer le dispositif de mesure (54) à l'équipement facultatif (56).

5. Système de mesure tel que défini dans la revendication 3, dans lequel le guide (50) peut être retiré de l'équipement facultatif (56) ou stocké dans celui-ci.

6. Système de mesure tel que défini dans la revendication 1, dans lequel le guide (50) est réalisé en un matériau ayant une élasticité de ressort.

7. Système de mesure tel que défini dans la revendication 1, dans lequel le guide (50) a l'espace (55) formé à partir d'une extrémité du dispositif de mesure (54).

8. Système de mesure tel que défini dans la revendication 4, dans lequel le guide (50) pousse l'extrémité ou la surface supérieure du dispositif de mesure (54).

9. Système de mesure tel que défini dans la revendication 3, dans lequel le guide (50) est amovible de l'équipement facultatif (56).
